# EUROPÄISCHE PATENTSCHRIFT

(11) **EP 3 561 045 B1**
(45) Veröffentlichungstag und Bekanntmachung des Hinweises auf die Patenterteilung: **13.01.2021**
(21) Anmeldenummer: 19170636.5
(22) Anmeldetag: 23.04.2019
(51) Int. Cl.: C12M 3/00, C12M 1/00, C12M 1/12

(54) **VERFAHREN ZUR KULTIVIERUNG UND DIFFERENZIERUNG VON ZELLEN**
METHOD FOR CULTURING AND DIFFERENTIATING CELLS
PROCÉDÉ DE CULTURE ET DE DIFFÉRENCIATION DE CELLULES

(30) Priorität: 24.04.2018 DE 102018206268
(43) Veröffentlichungstag der Anmeldung: 30.10.2019
(73) Patentinhaber: Fraunhofer-Gesellschaft zur Förderung der angewandten Forschung e.V., 80686 München (DE); Julius-Maximilians-Universität Würzburg, 97070 Würzburg (DE)
(72) Erfinder: EMMERT, Martin, 81669 München (DE); SOMOROWSKY, Ferdinand, 97082 Würzburg (DE); HEINRICH, Doris, 97082 Würzburg (DE)
(74) Vertreter: Schrell, Andreas

(56) Entgegenhaltungen:
- EP-A1- 1 382 669
- WO-A1-2005/011844
- WO-A1-2008/101011
- WO-A2-2009/128892
- DE-A1-102010 012 252
- DE-A1-102014 102 055
- US-A1- 2013 052 735
- ARMIN LENHART, RALF LÖSEL: "BioGlas - Scaffolds aus Glas für 'Tissue Engineering mit einstellbaren Wachstumsfaktoren", VORLAUFFORSCHUNG SCHRIFTREIHE, Bd. 2016/2017, 31. Dezember 2017 (2017-12-31), Seiten 58-67, XP002794243, Nürnberg
- DATABASE WPI Week 201769 Thomson Scientific, London, GB; AN 2017-632357 XP002794244, & CN 107 129 157 A (UNIV ZHEJIANG SCI-TECH) 5. September 2017 (2017-09-05)

## Beschreibung

Die vorliegende Erfindung betrifft ein Verfahren zur Kultivierung von Zellen auf einem Zellkultursubstrat, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist, und die Verwendung eines Zellkultursubstrats zur Kultivierung oder Differenzierung von Zellen, als Boden eines Zellkulturgefäßes oder Bioreaktors, als herausnehmbarer Einsatz für Zellkulturgefäße oder Bioreaktoren und/oder als perfusive Membran für 3D-Zellkultur-Reaktoren, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

Das Verhalten lebender Zellen in den komplexen dreidimensionalen Umgebungen von Geweben und Organen unterscheidet sich stark von dem Verhalten von Zellen auf konventionellen zweidimensionalen Kulturoberflächen aus Polystyrol oder silikatbasierten Gläsern, die standardmäßig als Kulturoberflächen für *in vitro-*Untersuchungen in der Medizinprodukte- und Pharmaindustrie zum Einsatz kommen. Aufgrund dieses unterschiedlichen Verhaltens der zu untersuchenden Zellen lassen sich experimentelle Ergebnisse, die mit Hilfe von gewöhnlichen zweidimensionalen Zellkultursystemen gewonnen wurden, nur bedingt auf den lebenden Organismus übertragen. Deshalb ist die möglichst realistische Simulation der physiologischen Bedingungen im menschlichen oder tierischen Körper eine besondere Herausforderung bei der Kultivierung von Zellen.

Um eine möglichst realistische Simulation der in Geweben und Organen des menschlichen oder tierischen Körpers vorliegenden Zellumgebung zu erreichen, werden im Stand der Technik unter anderem dreidimensionale Zellkultursysteme auf Grundlage von Matrigel oder Sphäroiden eingesetzt. Die verwendeten biologisch gewonnenen/hergestellten dreidimensionalen Zellkultivierungssysteme haben insbesondere für die Nutzung in groß angelegten High-Throughput-Studien entscheidende Nachteile gegenüber konventionellen zweidimensionalen Zellkultursystemen. So kommt es durch die biologische Herstellung der dreidimensionalen Zellkultivierungssysteme zu einer unerwünschten Variabilität. Zudem ist die Kultivierung von Zellen in dreidimensionalen Zellkultursystemen mit einem erheblich höheren Arbeitsaufwand und deutlich höheren Kosten verbunden. Weitere Nachteile liegen in der begrenzten Lagerkapazität und -stabilität der bislang verfügbaren dreidimensionalen biologischen Zellkultursysteme und der schlechten Mikroskopierbarkeit derartiger Zellkulturen. Konventionelle zweidimensionale Zellkultursysteme hingegen zeichnen sich bekanntermaßen insbesondere durch eine standardisierte, einfache Handhabung, die Autoklavier- bzw. Sterilisierbarkeit der Systeme, eine aufgrund der planaren Kulturoberfläche homogene Zellbesiedelung, eine gute Mikroskopierbarkeit und die Möglichkeit der Vorproduktion und Lagerung solcher Systeme in großem Maßstab aus.

Eine Möglichkeit, ein möglichst physiologisches Zellverhalten in zweidimensionalen Zellkultursystemen zu erreichen, besteht darin, dem Kulturmedium Zytokine oder andere Additive hinzuzufügen, um die Zellen beispielsweise zur Migration anzuregen oder deren Differenzierung in eine bestimmte Richtung zu initiieren. Dieses Verfahren führt durch den Einsatz von spezifisch angepassten Kulturmedien zu einem erheblichen Kostenaufwand und hat zudem den Nachteil, dass die Differenzierung der Zellen in Standardgefäßen aufgrund der glatten Oberfläche ohne jeglichen topographischen Stimulus erfolgt und somit das Verhalten einer solchen Zellkultur nur bedingt auf das Verhalten von Zellen im menschlichen oder tierischen Körpers übertragbar ist.

Um ein möglichst realistisches Adhäsionsverhalten von Zellen in zweidimensionalen Systemen zu gewährleisten, werden im Stand der Technik Zellkulturgefäße eingesetzt, die zur verbesserten Adhäsion von Proteinen auf der Oberfläche mittels Plasma- oder Corona-Behandlungen hydrophilisiert wurden, oder solche, deren Oberfläche zu diesem Zweck direkt mit einem die Zelladhäsion vermittelnden Protein, wie Fibronectin, Vitronectin oder Poly-L-Lysin, beschichtet wurden. Derartig beschichtete Zellkulturgefäße weisen jedoch nachteiligerweise nur eine sehr begrenzte Lagerstabilität auf.

DE 10 2014 102055 offenbart ein Verfahren und eine Vorrichtung zur Kultivierung von Zellen auf einem Substratträger aus Glas, dessen nano- und mikroporöse Oberfläche funktionalisiert ist.

Aufgrund des Dilemmas zwischen einfacher Handhabung von zweidimensionalen Zellkultursystemen einerseits und dem physiologischen Zellverhalten in dreidimensionalen Zellkultursystemen andererseits besteht ein großer Bedarf an Systemen, die die Vorteile von zweidimensionalen Zellkultursystemen mit einem möglichst physiologischen Zellverhalten vereinen und vorteilhafterweise in bestehende Standard-Laborgeräte und High-Throughput-Prozesse integrierbar sind.

Es ist daher das der vorliegenden Erfindung zugrunde liegende technische Problem, die vorgenannten Nachteile im Stand der Technik zu überwinden, insbesondere durch die Bereitstellung eines Verfahrens zur Kultivierung und/oder zur Differenzierung von Zellen, insbesondere Stammzellen, wobei das Verfahren eine einfache Handhabung der Zellkulturen und gleichzeitig eine möglichst realistische Simulation physiologischen Zellverhaltens erlaubt.

Die vorliegende Erfindung löst das ihr zugrundeliegende Problem insbesondere durch die technische Lehre der unabhängigen Ansprüche.

Dabei betrifft die vorliegende Erfindung ein Verfahren zur Kultivierung von Zellen, umfassend die Schritte:
a) Bereitstellen mindestens einer in einem Zellkulturmedium vorliegenden Zelle und eines Zellkultursubstrats,
b) Kontaktieren der mindestens einen in dem Zellkulturmedium vorliegenden Zelle mit dem Zellkultursubstrat,
c) Inkubieren der mindestens einen in dem Zellkulturmedium vorliegenden Zelle auf dem Zellkultursubstrat,
dadurch gekennzeichnet, dass das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

In besonders vorteilhafter Weise ermöglicht es das Verfahren zur Kultivierung von Zellen gemäß der vorliegenden Erfindung, die Vorteile von zweidimensionalen Zellkultursystemen mit denen von dreidimensionalen Zellkultursystemen zu vereinen und somit insbesondere eine möglichst realistische Simulation des physiologischen Verhaltens von Zellen mit einer einfachen Handhabung zu kombinieren. Dabei führt insbesondere die Verwendung eines Zellkultursubstrats, das ein Zellkultursubstrat aus Glas umfasst, wobei mindestens ein Teil des Zellkultursubstrats aus Glas eine nanoporös strukturierte Oberfläche mit einem durchschnittlichen Porendurchmesser von 2 bis 150 nm aufweist, zu einer topographischen Stimulierung der Zellen und erlaubt gleichzeitig die Nutzung der Arbeitsschritte und Medien von konventionellen zweidimensionalen Kulturen. Das erfindungsgemäß verwendete, ein Zellkultursubstrat aus Glas umfassende Zellkultursubstrat weist demgemäß zumindest in einem Teil der Oberfläche des Zellkultursubstrats aus Glas eine intrinsische Nanostrukturierung auf, so dass im Gegensatz zu den im Stand der Technik bekannten Zellkultursubstraten keine nachträgliche, aktive Strukturierung erfolgen muss, um eine nanoporös strukturierte Oberfläche zu erhalten, was unter anderem zu einer deutlichen Reduzierung der Produktionskosten führt. Mit Hilfe des erfindungsgemäßen Verfahrens ist es zudem durch die Kultivierung von in einem Zellkulturmedium vorliegenden Zellen auf einem Zellkultursubstrat aus Glas, wobei mindestens ein Teil des Zellkultursubstrats aus Glas eine nanoporös strukturierte Oberfläche mit einem durchschnittlichen Porendurchmesser von 2 bis 150 nm aufweist, vorteilhafterweise möglich, durch geeignete Wahl eines definierten durchschnittlichen Porendurchmessers im Bereich von 2 bis 150 nm bestimmte Zellfunktionen unterschiedlicher Zelltypen gezielt zu unterstützen oder zu steuern.

In einer weiteren bevorzugten Ausführungsform der vorliegenden Erfindung handelt es sich bei der mindestens einen in Schritt a) bereitgestellten in einem Zellkulturmedium vorliegenden Zelle um eine Stammzelle und bei dem Verfahren zur Kultivierung von Zellen um ein Verfahren zur Differenzierung von Stammzellen. Durch die Bereitstellung mindestens einer in einem Zellkulturmedium vorliegenden Stammzelle in Schritt a), das Kontaktieren der mindestens einen in einem Zellkulturmedium vorliegenden Stammzelle mit dem Zellkultursubstrat in Schritt b) und das Inkubieren der mindestens einen in einem Zellkulturmedium vorliegenden Stammzelle auf dem Zellkultursubstrat in Schritt c), wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist, ist es vorteilhafterweise möglich, eine Differenzierung der Stammzellen ohne Zugabe von Additiven zu initiieren. Dabei wirkt die nanoporös strukturierte Oberfläche mit einem durchschnittlichen Porendurchmesser von 2 bis 150 nm des Zellkultursubstrats aus Glas als die Differenzierung der Zellen initiierender topographischer Stimulus.

In besonders bevorzugter Ausführungsform besteht das Verfahren gemäß der vorliegenden Erfindung aus den Verfahrensschritten a) bis c), das heißt vor, nach und/oder zwischen den Verfahrensschritten a), b) und c) finden keine weiteren Verfahrensschritte statt. In bevorzugter Ausführungsform wird das Verfahren in der Reihenfolge der Verfahrensschritte a), b) und c) durchgeführt.

In einer bevorzugten Ausführungsform der vorliegenden Erfindung besteht das Zellkultursubstrat vollständig aus Glas, wobei das Glas eine nanoporös strukturierte Oberfläche mit einem durchschnittlichen Porendurchmesser von 2 bis 150 nm aufweist. In einer weiteren bevorzugten Ausführungsform der vorliegenden Erfindung besteht das Zellkultursubstrat vollständig aus Glas, wobei mindestens ein Teil des Zellkultursubstrats aus Glas eine nanoporös strukturierte Oberfläche mit einem durchschnittlichen Porendurchmesser von 2 bis 150 nm aufweist.

In einer bevorzugten Ausführungsform der vorliegenden Erfindung besteht das Zellkultursubstrat zu mindestens 20 %, bevorzugt mindestens 25 %, bevorzugt mindestens 30 %, bevorzugt zu mindestens 35 %, bevorzugt mindestens 40 %, bevorzugt mindestens 45 %, bevorzugt zu mindestens 55 %, bevorzugt mindestens 60 %, bevorzugt mindestens 65 %, bevorzugt zu mindestens 70 %, bevorzugt mindestens 75 %, bevorzugt mindestens 80 %, bevorzugt zu mindestens 85 %, bevorzugt mindestens 90 %, bevorzugt mindestens 95 %, bevorzugt zu mindestens 98 %, aus Glas.

Erfindungsgemäß weist mindestens ein Teil des Zellkultursubstrats aus Glas, bevorzugt weisen mindestens 0,1 %, bevorzugt mindestens 0,5 %, bevorzugt mindestens 1 %, bevorzugt mindestens 2 %, bevorzugt mindestens 3 %, bevorzugt mindestens 4 %, bevorzugt mindestens 5 %, bevorzugt mindestens 10 %, bevorzugt mindestens 15 %, bevorzugt mindestens 20 %, bevorzugt mindestens 25 %, bevorzugt mindestens 30 %, bevorzugt mindestens 35 %, bevorzugt mindestens 40 %, bevorzugt mindestens 45 %, bevorzugt mindestens 55 %, bevorzugt mindestens 60 %, bevorzugt mindestens 65 %, bevorzugt mindestens 70 %, bevorzugt mindestens 75 %, bevorzugt mindestens 80 %, bevorzugt mindestens 85 %, bevorzugt mindestens 90 %, bevorzugt mindestens 95 %, bevorzugt mindestens 96 %, bevorzugt mindestens 97 %, bevorzugt mindestens 98 %, bevorzugt mindestens 99 %, bevorzugt 100 % des Zellkultursubstrats aus Glas, eine nanoporös strukturierte Oberfläche mit einem durchschnittlichen Porendurchmesser von 2 bis 150 nm auf.

In einer weiteren bevorzugten Ausführungsform der vorliegenden Erfindung ist die nanoporös strukturierte Oberfläche mit einem durchschnittlichen Porendurchmesser von 2 bis 150 nm auf dem Zellkultursubstrat aus Glas als Array, bevorzugt als Microarray, ausgebildet. Bei einem solchen Array, bevorzugt Microarray, handelt es sich bevorzugt um auf dem Zellkultursubstrat aus Glas in einem, bevorzugt regelmäßigen, Abstand voneinander angeordnete, bevorzugt kreisförmige oder rechteckige, insbesondere quadratische, Bereiche, die eine nanoporös strukturierte Oberfläche mit einem durchschnittlichen Porendurchmesser von 2 bis 150 nm aufweisen.

In einer bevorzugten Ausführungsform der vorliegenden Erfindung weist die nanoporös strukturierte Oberfläche des Zellkultursubstrats, insbesondere des Zellkultursubstrats aus Glas, einen durchschnittlichen Porendurchmesser von 3 bis 150 nm, bevorzugt 4 bis 150 nm, bevorzugt 5 bis 150 nm, bevorzugt 10 bis 150 nm, bevorzugt 20 bis 150 nm, bevorzugt 30 bis 150 nm, bevorzugt 40 bis 150 nm, bevorzugt 50 bis 150 nm, bevorzugt 60 bis 150 nm, bevorzugt 70 bis 150 nm, bevorzugt 80 bis 150 nm, auf.

Besonders bevorzugt weist die nanoporös strukturierte Oberfläche des Zellkultursubstrats, insbesondere des Zellkultursubstrats aus Glas, einen durchschnittlichen Porendurchmesser von 60 bis 140 nm, bevorzugt 70 bis 135 nm, bevorzugt 75 bis 130 nm, bevorzugt 80 bis 125 nm, auf.

In einer bevorzugten Ausführungsform des Verfahrens zur Differenzierung von Zellen, insbesondere Stammzellen, gemäß der vorliegenden Erfindung werden dem Zellkulturmedium keine Additive, insbesondere keine Zytokine, zugesetzt.

In einer weiteren bevorzugten Ausführungsform des Verfahrens zur Differenzierung von Zellen, insbesondere Stammzellen, gemäß der vorliegenden Erfindung werden dem Zellkulturmedium Additive, wie Zytokine, zugesetzt. Gemäß dieser bevorzugten Ausführungsform ist es vorteilhafterweise möglich, die Differenzierung von Zellen, insbesondere Stammzellen, mit einer gegenüber den im Stand der Technik bekannten Verfahren zur Differenzierung von Zellen, insbesondere Stammzellen, verringerten Konzentration an Additiven, wie Zytokinen, zu erreichen. Zudem ist es gemäß dieser bevorzugten Ausführungsform vorteilhafterweise möglich, eine beschleunigte Differenzierung von Zellen, insbesondere Stammzellen, gegenüber den im Stand der Technik bekannten Verfahren zur Differenzierung von Zellen, insbesondere Stammzellen, zu erreichen.

In einer bevorzugten Ausführungsform des Verfahrens zur Kultivierung von Zellen, insbesondere des Verfahrens zur Differenzierung von Stammzellen, weist das Zellkultursubstrat, insbesondere das Zellkultursubstrat aus Glas, eine Dicke von 10 bis 5000 µm, bevorzugt 20 bis 5000 µm, bevorzugt 30 bis 4500 µm, bevorzugt 40 bis 4000 µm, bevorzugt 50 bis 4000 µm, bevorzugt 60 bis 3500 µm, bevorzugt 70 bis 3000 µm, bevorzugt 80 bis 3000 µm, bevorzugt 90 bis 2500 µm, bevorzugt 100 bis 2000 µm, bevorzugt 150 bis 2000 µm, bevorzugt 200 bis 1500 µm, bevorzugt 220 bis 1000 µm, bevorzugt 240 bis 980 µm, bevorzugt 260 bis 960 µm, bevorzugt 280 bis 940 µm, bevorzugt 300 bis 920 µm, bevorzugt 320 bis 900 µm, bevorzugt 340 bis 880 µm, bevorzugt 360 bis 860 µm, bevorzugt 380 bis 840 µm, bevorzugt 400 bis 820 µm, bevorzugt 420 bis 800 µm, bevorzugt 440 bis 780 µm, bevorzugt 460 bis 760 µm, bevorzugt 480 bis 740 µm, bevorzugt 500 bis 720 µm, bevorzugt 500 bis 700 µm, auf. Bevorzugt ist das Zellkultursubstrat, insbesondere das Zellkultursubstrat aus Glas, eine Membran.

In einer bevorzugten Ausführungsform der vorliegenden Erfindung ist das Zellkultursubstrat, insbesondere das Zellkultursubstrat aus Glas, ein poröses Glas, bevorzugt VYCOR-Glas. Besonders bevorzugt ist das Zellkultursubstrat, insbesondere das Zellkultursubstrat aus Glas, ein poröses Glas, bevorzugt ein VYCOR-Glas, das nach dem in der US 2,106,744 beschriebenen Verfahren hergestellt ist. Bevorzugt ist das Zellkultursubstrat, insbesondere das Zellkultursubstrat aus Glas, ein poröses Glas, bevorzugt VYCOR-Glas, das durch Extraktion, insbesondere durch Auslaugen, aus phasengetrenntem Alkaliborosilikatglas hergestellt ist.

In einer weiteren bevorzugten Ausführungsform der vorliegenden Erfindung ist das Zellkultursubstrat, insbesondere das Zellkultursubstrat aus Glas, ein Glas, dessen nanoporös strukturierte Oberfläche durch teilweise, bevorzugt vollständige Extraktion, insbesondere durch teilweises, bevorzugt vollständiges Auslaugen, aus phasengetrenntem Alkaliborosilikatglas hergestellt ist. Durch die teilweise Extraktion, insbesondere durch teilweises Auslaugen, aus phasengetrenntem Alkaliborosilikatglas ist es möglich, ein Zellkultursubstrat, insbesondere ein Zellkultursubstrat aus Glas, zu erhalten, bei dem lediglich die Oberfläche des Glases eine Nanostrukturierung mit einem durchschnittlichen Porendurchmesser von 2 bis 150 nm aufweist.

In einer bevorzugten Ausführungsform der vorliegenden Erfindung besteht das Zellkultursubstrat, insbesondere das Zellkultursubstrat aus Glas, vor dem teilweisen oder vollständigen Auslaugen aus 30 bis 80 Gew.-% Siliziumdioxid (SiO₂), 20 bis 70 Gew.-% Boroxid (B₂O₃) und 5 bis 20 Gew.-% Natriumoxid (Na₂O), bevorzugt aus 70 Gew.-% SiO₂, 23 Gew.-% B₂O₃ und 7 Gew.-% Na₂O.

In einer weiteren bevorzugten Ausführungsform der vorliegenden Erfindung besteht das Zellkultursubstrat, insbesondere das Zellkultursubstrat aus Glas, vor dem teilweisen oder vollständigen Auslaugen aus 50 bis 80 Gew.-% Siliziumdioxid (SiO₂), 20 bis 45 Gew.-% Boroxid (B₂O₃) und 5 bis 20 Gew.-% Natriumoxid (Na₂O).

In einer bevorzugten Ausführungsform der vorliegenden Erfindung besteht das Zellkultursubstrat, insbesondere das Zellkultursubstrat aus Glas, insbesondere nach teilweisem oder vollständigem Auslaugen, aus 95 bis 98 Gew.-% SiO₂, 2,5 bis 3,5 Gew.-% B₂O₃ und 0,3 bis 0,6 Gew.-% Na₂O. Bevorzugt weist das Zellkultursubstrat, insbesondere das Zellkultursubstrat aus Glas, nach dem teilweisen oder vollständigen Auslaugen mindestens 95 Gew.-% SiO₂, bevorzugt mindestens 95,5 Gew.-% SiO₂, bevorzugt mindestens 96 Gew.-% SiO₂, auf.

In einer bevorzugten Ausführungsform der vorliegenden Erfindung besitzt das Zellkultursubstrat, insbesondere das Zellkultursubstrat aus Glas, insbesondere nach teilweisem oder vollständigem Auslaugen, eine Porosität von 20 bis 70 %, bevorzugt 21 bis 68 %, bevorzugt 21 bis 66 %, bevorzugt 22 bis 64 %, bevorzugt 22 bis 62 %, bevorzugt 23 bis 60 %, bevorzugt 23 bis 58 %, bevorzugt 24 bis 56 %, bevorzugt 24 bis 54 %, bevorzugt 25 bis 52 %, bevorzugt 25 bis 50 %, bevorzugt 25 bis 48 %, bevorzugt 26 bis 46 %, bevorzugt 26 bis 44 %, bevorzugt 27 bis 43 %, bevorzugt 28 bis 42 %, bevorzugt 29 bis 41 %, bevorzugt 30 bis 40 %, bevorzugt 31 bis 39 %, bevorzugt 32 bis 38 %, bevorzugt 33 bis 37 %, bevorzugt 34 bis 36 %, bevorzugt 35 %.

In einer bevorzugten Ausführungsform der vorliegenden Erfindung beträgt die Oberfläche des eine nanoporös strukturierte Oberfläche aufweisenden Zellkultursubstrats aus Glas 10 bis 2000 m²/g, bevorzugt 15 bis 1500 m²/g, bevorzugt 20 bis 1000 m²/g, bevorzugt 20 bis 500 m²/g, bevorzugt 50 bis 400 m²/g, bevorzugt 60 bis 480 m²/g, bevorzugt 70 bis 460 m²/g, bevorzugt 80 bis 440 m²/g, bevorzugt 90 bis 420 m²/g, bevorzugt 100 bis 400 m²/g, bevorzugt 100 bis 350 m²/g, bevorzugt 100 bis 300 m²/g, bevorzugt 120 bis 280 m²/g, bevorzugt 140 bis 260 m²/g, bevorzugt 160 bis 240 m²/g.

In einer bevorzugten Ausführungsform der vorliegenden Erfindung ist das Zellkultursubstrat, insbesondere das Zellkultursubstrat aus Glas, transparent. In einer weiteren bevorzugten Ausführungsform ist das Zellkultursubstrat, insbesondere das Zellkultursubstrat aus Glas, opak.

In einer bevorzugten Ausführungsform der vorliegenden Erfindung weist das Zellkultursubstrat, insbesondere das Zellkultursubstrat aus Glas, keine gerichtete Oberflächenstruktur auf.

In einer weiteren bevorzugten Ausführungsform der vorliegenden Erfindung weist das Zellkultursubstrat, insbesondere das Zellkultursubstrat aus Glas, keine Oberflächenbeschichtung und/oder Oberflächenfunktionalisierung auf.

In einer weiteren bevorzugten Ausführungsform der vorliegenden Erfindung weist das Zellkultursubstrat, insbesondere das Zellkultursubstrat aus Glas, eine Oberflächenbeschichtung und/oder Oberflächenfunktionalisierung auf.

In einer weiteren bevorzugten Ausführungsform der vorliegenden Erfindung ist die mindestens eine Zelle eine Stammzelle, insbesondere eine humane Stammzelle. Bevorzugt ist die mindestens eine Stammzelle eine humane mesenchymale Stammzelle (hMSC), bevorzugt eine primäre humane mesenchymale Stammzelle. Bevorzugt ist die mindestens eine Zelle, insbesondere Stammzelle, eine iPS-Zelle (induzierte pluripotente Stammzelle), insbesondere eine humane iPS-Zelle (hiPS).

In einer weiteren bevorzugten Ausführungsform der vorliegenden Erfindung ist die mindestens eine Zelle eine Tumorzelle. Bevorzugt ist die mindestens eine Tumorzelle eine humane Tumorzelle, bevorzugt eine primäre humane Tumorzelle.

In einer weiteren bevorzugten Ausführungsform der vorliegenden Erfindung ist die mindestens eine Zelle eine Zelle einer Tumorzelllinie. Bevorzugt ist die mindestens eine Tumorzelle eine Zelle einer humanen Tumorzelllinie, die für die Verwendung in Medikamententests geeignet ist.

In einer weiteren bevorzugten Ausführungsform der vorliegenden Erfindung ist die mindestens eine Zelle ein Fibroblast. Bevorzugt ist die mindestens eine Zelle eine Zelle einer humanen Fibroblastenzelllinie, die für die Verwendung in Standard-Zytotoxizitätstests geeignet ist.

In einer bevorzugten Ausführungsform der vorliegenden Erfindung handelt es sich bei dem Zellkultursubstrat um einen Teil eines Zellkulturgefäßes oder Bioreaktors, bevorzugt um den Boden eines Zellkulturgefäßes oder Bioreaktors. In einer bevorzugten Ausführungsform der vorliegenden Erfindung handelt es sich bei dem Zellkultursubstrat um eine auf den Boden eines Zellkulturgefäßes oder Bioreaktors aufgebrachte, bevorzugt aufgeschweißte oder ausgesinterte, Membran. In einer weiteren bevorzugten Ausführungsform der vorliegenden Erfindung handelt es sich bei dem Zellkultursubstrat um eine in das Zellkulturgefäß oder in den Bioreaktor integrierte Membran.

In einer bevorzugten Ausführungsform der vorliegenden Erfindung handelt es sich bei dem Zellkultursubstrat um einen Einsatz für Zellkulturgefäße oder Bioreaktoren, bevorzugt um eine in das Zellkulturgefäß oder in den Bioreaktor einsetzbare Membran. Dabei kann das Zellkultursubstrat jegliche als Einsatz für Zellkulturgefäße oder Bioreaktoren geeignete Form aufweisen.

Die vorliegende Erfindung betrifft zudem die Verwendung eines Zellkultursubstrats zur Kultivierung und/oder Differenzierung von Zellen, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

Des Weiteren betrifft die vorliegende Erfindung die Verwendung eines Zellkultursubstrats als Boden eines Zellkulturgefäßes oder eines Bioreaktors, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

Zudem betrifft die vorliegende Erfindung die Verwendung eines Zellkultursubstrats als herausnehmbarer Einsatz eines Zellkulturgefäßes oder eines Bioreaktors, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

Die vorliegende Erfindung betrifft auch die Verwendung eines Zellkultursubstrats als perfusive Membran für 3D-Zellkultur-Reaktoren, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

Die im Zusammenhang mit dem erfindungsgemäßen Verfahren zur Kultivierung von Zellen offenbarten Ausführungsformen gelten mutatis mutandis auch für die Verwendung eines Zellkultursubstrats aus Glas.

Im Zusammenhang mit der vorliegenden Erfindung wird unter dem Begriff "Zellkultursubstrat" ein Material verstanden, auf dem ein Wachstum von Zellen stattfinden kann. Dabei umfasst das "Zellkultursubstrat" gemäß der vorliegenden Erfindung ein Zellkultursubstrat aus Glas, wobei mindestens ein Teil dieses Zellkultursubstrats aus Glas eine nanoporös strukturierte Oberfläche mit einem durchschnittlichen Porendurchmesser von 2 bis 150 nm aufweist. Das heißt, von dem Begriff "Zellkultursubstrat" sind Ausführungsformen erfasst, bei denen das gesamte Zellkultursubstrat aus Glas besteht und mindestens ein Teil dieses Zellkultursubstrats aus Glas, bevorzugt das gesamte Zellkultursubstrat aus Glas, eine nanoporös strukturierte Oberfläche mit einem durchschnittlichen Porendurchmesser von 2 bis 150 nm aufweist. Andererseits sind von dem Begriff auch Ausführungsformen erfasst, bei denen das Zellkultursubstrat gemäß der vorliegenden Erfindung aus verschiedenen Materialien besteht, wobei mindestens ein Teil des Zellkultursubstrats aus Glas besteht, wovon wiederum mindestens ein Teil eine nanoporös strukturierte Oberfläche mit einem durchschnittlichen Porendurchmesser von 2 bis 150 nm besitzt. Denkbar sind in diesem Zusammenhang beispielsweise auch Ausführungsformen, bei denen nur bestimmte Bereiche des Zellkultursubstrats aus Glas eine nanoporös strukturierte Oberfläche mit einem durchschnittlichen Porendurchmesser von 2 bis 150 nm aufweisen und andere Bereiche des Zellkultursubstrats aus Glas keine derartige nanoporös strukturierte Oberfläche besitzen.

Im Zusammenhang mit der vorliegenden Erfindung wird unter dem Begriff "intrinsische Nanostrukturierung" des Zellkultursubstrats verstanden, dass zumindest ein Teil des Zellkultursubstrats aus Glas eine nanoporös strukturierte Oberfläche aufweist, also eine Oberfläche mit Poren mit einem durchschnittlichen Porendurchmesser von 2 bis 150 nm, insbesondere von 3 bis 150 nm, bevorzugt 4 bis 150 nm, bevorzugt 5 bis 150 nm, bevorzugt 10 bis 150 nm, bevorzugt 20 bis 150 nm, bevorzugt 30 bis 150 nm, bevorzugt 40 bis 150 nm, bevorzugt 50 bis 150 nm, bevorzugt 60 bis 150 nm, bevorzugt 70 bis 150 nm, bevorzugt 80 bis 150 nm.

Unter dem Begriff "und/oder" wird in Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind.

Im Zusammenhang mit der vorliegenden Erfindung wird unter dem Begriff "umfassend" verstanden, dass zusätzlich zu den von dem Begriff explizit erfassten Elementen noch weitere, nicht explizit genannte Elemente hinzutreten können. Im Zusammenhang mit der vorliegenden Erfindung wird unter diesem Begriffen auch verstanden, dass allein die explizit genannten Elemente erfasst werden und keine weiteren Elemente vorliegen. In dieser besonderen Ausführungsform ist die Bedeutung des Begriffes "umfassend" gleichbedeutend mit dem Begriff "bestehend aus". Darüber hinaus erfasst der Begriff "umfassend" auch Gesamtheiten, die neben den explizit genannten Elementen auch weitere nicht genannte Elemente enthalten, die jedoch von funktionell und qualitativ unter- oder nebengeordneter Natur sind. In dieser Ausführungsform ist der Begriff "umfassend" gleichbedeutend mit dem Begriff "im Wesentlichen bestehend aus".

Weitere bevorzugte Ausführungsformen ergeben sich aus den Unteransprüchen.

Die vorliegende Erfindung wird anhand der nachfolgenden Beispiele und dazugehörigen Figuren illustriert.
Figur 1 zeigt die relative Expression der knorpelspezifischen Gene Col1a1 (Fig. 1a), Col10 (Fig. 1b) und Sox9 (Fig. 1c) in primären humanen mesenchymalen Stammzellen (hMSCs) von zwei Patienten auf zwei Kontrolloberflächen (TCPS = Tissue Culture Polysterene, FG = flaches Deckglas) nach 7 bis 12 Tagen im Vergleich zum Wachstum der Zellen auf dem Zellkultursubstrat gemäß der vorliegenden Erfindung (durchschnittlicher Porendurchmesser 17 nm, Balken repräsentieren die Mittelwerte der beiden Patienten).
Figur 2 zeigt eine Phalloidin-Färbung des Aktin-Zytoskeletts von auf einer nanoporösen Glasmembran mit einem durchschnittlichen Porendurchmesser von 17 nm gewachsenen primären humanen mesenchymalen Stammzellen (hMSCs) (links) und der auf den beiden Kontrollsubstraten gewachsenen Zellen (mitte, rechts) nach 1, 2, 5 und 7 Tagen.
Figur 3 zeigt Proliferationsraten von L929 Fibroblasten innerhalb definierter Zeiträume auf Zellkultursubstraten gemäß der vorliegenden Erfindung mit unterschiedlichen durchschnittlichen Porendurchmessern und auf zwei Kontrolloberflächen, jeweils unter Standard-Zellkulturbedingungen (TCPS = Tissue Culture Polysterene, FG = flaches Deckglas).
Figur 4a zeigt die Entwicklung der relativen Zellzahl von SK-MEL-28 Melanomzellen in Überkopfkultur auf Zellkultursubstraten gemäß der vorliegenden Erfindung mit einem durchschnittlichen Porendurchmesser von 20 nm und auf flachen Deckgläsern (FG).
Figur 4b zeigt die Adhäsion von SK-MEL-28 Melanomzellen auf einer nanoporösen Glasmembran mit einem durchschnittlichen Porendurchmesser von 20 nm und auf einer flachen unporösen Glasoberfläche (FG) nach 3 Stunden Inkubation auf dem jeweiligen Substrat.
Figur 5 zeigt schematisch die Morphologie und die Adhäsion von auf einer nanoporösen Glasmembran gewachsenen SK-MEL-28 Melanomzellen im Vergleich zu auf einer flachen unporösen Glasoberfläche (FG) gewachsenen SK-MEL-28 Melanomzellen zu unterschiedlichen Zeitpunkten in Überkopfkultur.
Figur 6 zeigt die Analyse der mRNA-Expression von L929 Zellen nach 48 h Kultivierung auf den unterschiedlichen nanoporösen Glasmembranen (17 nm, 45 nm, 81 nm, 124 nm) und auf den beiden Kontrolloberflächen (FG, TCPS).
Figur 7 zeigt die Entwicklung der relativen Zellzahl von MDA-MB-231 Brustkrebszellen in Überkopfkultur auf Zellkultursubstraten gemäß der vorliegenden Erfindung mit unterschiedlichem durchschnittlichen Porendurchmesser (17 nm, 26 nm, 46 nm, 81 nm, 124 nm) und auf flachen Deckgläsern (FG) ohne Wirkstoff (CONTROL) und unter Einfluss von jeweils 500 nM Paclitaxel (TREATMENT).

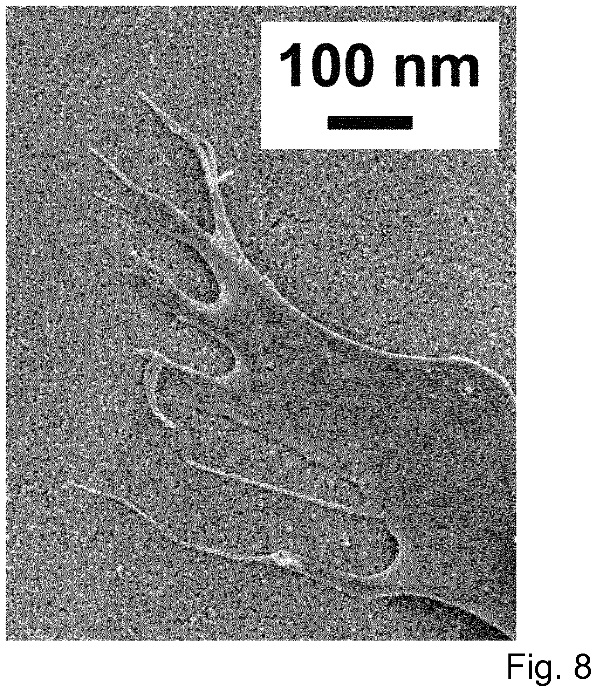

Figur 8 zeigt eine rasterelektronenmikroskopische Aufnahme eines Lamellopodiums einer humanen mesenchymalen Stammzelle (hMSC) mit vielen kleinen Filopodien nach zwei Tagen Inkubation auf einem erfindungsgemäßen Zellkultursubstrat mit einem durchschnittlichen Porendurchmesser von 17 nm.

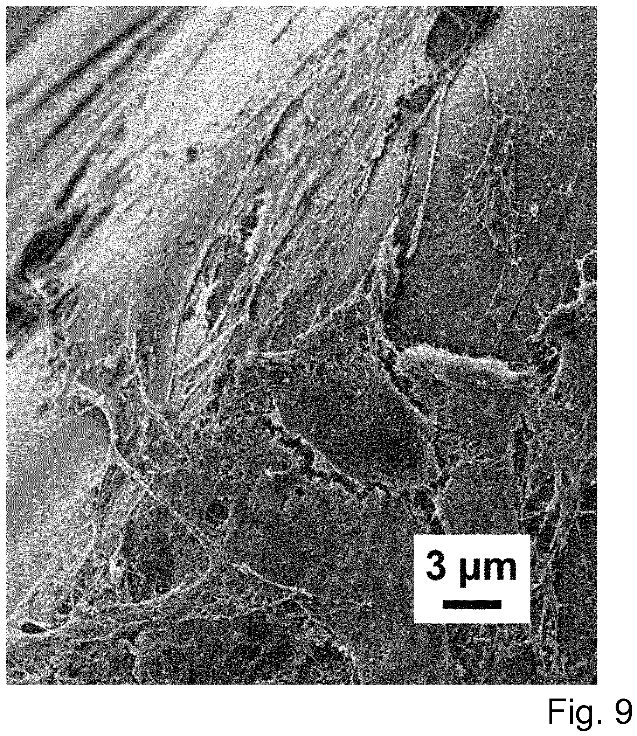

Figur 9 zeigt eine rasterelektronenmikroskopische Aufnahme von humanen mesenchymalen Stammzellen (hMSCs), die zwei Tage auf einem Zellkultursubstrat gemäß der vorliegenden Erfindung mit einem durchschnittlichen Porendurchmesser von 17 nm inkubiert wurden.

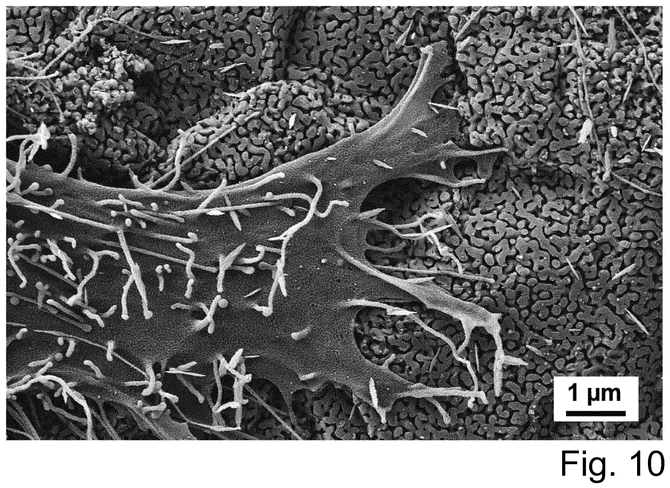

Figur 10 zeigt eine rasterelektronenmikroskopische Aufnahme eines Lamellopodiums eines L929 Fibroblasten mit vielen kleinen Filopodien nach zwei Tagen Inkubation auf einem erfindungsgemäßen Zellkultursubstrat mit einem durchschnittlichen Porendurchmesser von 124 nm.
Figur 11 zeigt vier unterschiedliche nanoporöse Glasmembranen gemäß der vorliegenden Erfindung (oben) und rasterelektronenmikroskopische Aufnahmen der nanoporösen Oberflächenstruktur der einzelnen Membranen.

### Beispiele:

### 1. Herstellung und physikalische Eigenschaften nanoporöser Glasmembranen mit unterschiedlicher Porengröße

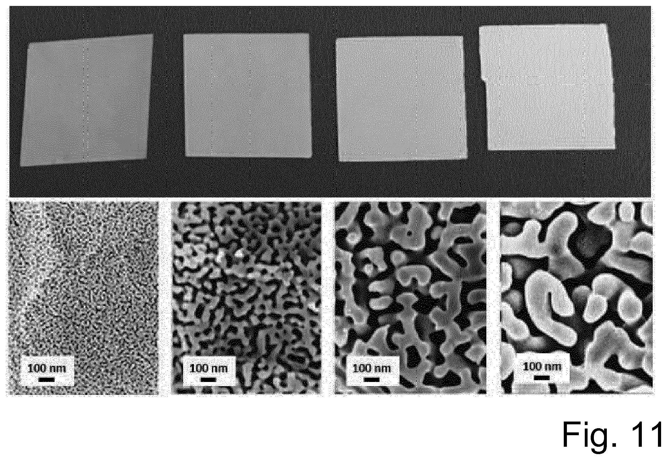

Um den Einfluss von nanoporösem Glas auf das Verhalten lebender Zellen zu untersuchen und die Abhängigkeit vom Porendurchmesser zu ermitteln wurden unter Verwendungen eines modifizierten VYCOR-Prozesses für die Beispiele 2 bis 7 Glasmembranen mit unterschiedlichem durchschnittlichen Porendurchmesser hergestellt. Dabei wurde festgestellt, dass die Membranen mit steigender Temperatur während des Entmischens zunehmend opak wurden, was auf einen vergrößerten durchschnittlichen Porendurchmesser hindeutet (Figur 11). Diese makroskopische Beobachtung konnte durch UV/VIS Experimente bestätigt werden, aus denen klar hervorgeht, dass es mit steigender Temperatur während des Entmischens zu einer Verbreiterung des Bereichs der vom nanoporösen Glas absorbierten Wellenlängen kommt. Durch die Regulierung der Temperatur während des Entmischens, des Abkühlungsprozesses und dem kontrollierten Auslaugen ist es möglich, nanoporöse Glasmembranen mit einem durchschnittlichen Porendurchmesser zwischen 17 und 124 nm und einer Dicke von nur 250 µm herzustellen.

### 2. Kultur und Induktion der chondrogenen Differenzierung von hMSCs

Zur Untersuchung der Fähigkeit der Zellkultursubstrate gemäß der vorliegenden Erfindung zur Induktion einer chondrogenen Differenzierung wurden primäre hMSCs zweier Patienten auf zwei Kontrolloberflächen (TCPS = Tissue Culture Polysterene, FG = flaches Deckglas) und auf einem Zellkultursubstrat gemäß der vorliegenden Erfindung inkubiert, nämlich einem Zellkultursubstrat, welches eine nanoporös strukturierte VYCOR-Membran mit einer durchschnittlichen Porengröße von 17 nm aufweist, und die relative Expression der knorpelspezifischen Gene Col1a1, Col10 und SOX9 mittels qPCR bestimmt.

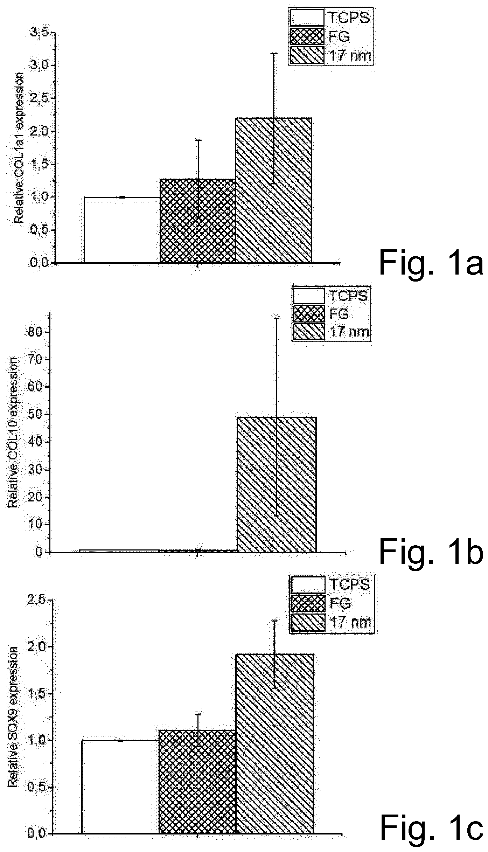

Im Vergleich zu den beiden Kontrolloberflächen ist bei Inkubation der Zellen auf einem Zellkultursubstrat gemäß der vorliegenden Erfindung ein deutlicher Anstieg der relativen Expression von Col1a1 (Figur 1a), Col10 (Figur 1b) und SOX9 (Figur 1c) zu erkennen.

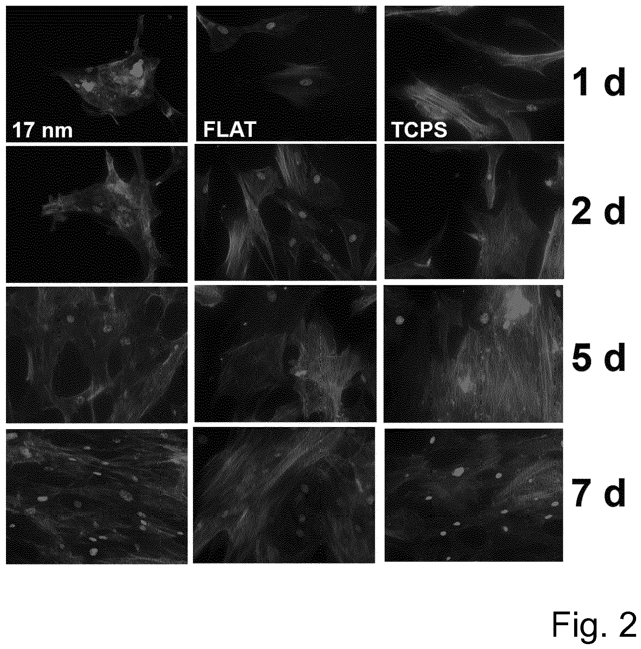

Zusätzlich wurde eine Phalloidin-Färbung des Aktin-Zytoskeletts der auf der nanoporösen Glasmembran mit einem durchschnittlichen Porendurchmesser von 17 nm gewachsenen Zellen und der auf den beiden Kontrollsubstraten gewachsenen Zellen durchgeführt. Es hat sich gezeigt, dass die Aktinfilamente in den auf den 2D Kontrolloberflächen kultivierten Zellen wesentlich geordneter vorliegen als die Aktinfilamente der auf nanoporösen Glasmembranen kultivierten Zellen (Figur 2).

Diese Induktion einer chondrogenen Differenzierung ohne externe Medienzusätze auf einem Zellkultursubstrat gemäß der vorliegenden Erfindung bereits nach der ersten Woche ermöglicht vorteilhafterweise die Nutzung von Zellkultursubstraten gemäß der vorliegenden Erfindung als Oberfläche zur schnellen und kostengünstigen Differenzierung von hMSCs.

### 3. Vergleich der Proliferationsraten von L929 Fibroblasten auf Zellkultursubstraten gemäß der vorliegenden Erfindung mit Proliferationsraten auf Kontrolloberflächen

Die Zellproliferation auf Standard-2D-Oberflächen weicht oft stark von der Proliferation im menschlichen Körper ab, da sich Zellen im Körper in 3D-Geweben befinden und oft vereinzelt proliferieren, während auf einer Standard-2D-Oberfläche ein meist unkontrolliertes Wachstum der Zellen möglich ist.

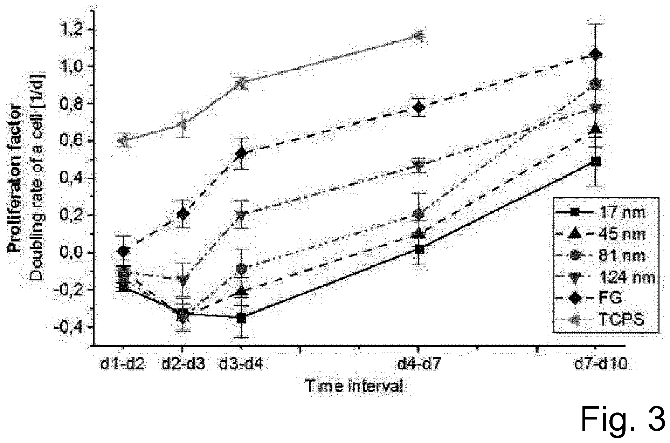

Im vorliegenden Versuch wurden L929 Fibroblasten unter Standard-2D-Kulturbedingungen auf zwei Kontrolloberflächen (TCPS = Tissue Culture Polysterene, FG = flaches Deckglas) und auf unterschiedlichen Zellkultursubstraten gemäß der vorliegenden Erfindung ausgesät und inkubiert, nämlich Zellkultursubstraten, welche jeweils eine nanoporös strukturierte VYCOR-Membran mit verschiedenen durchschnittlichen Porendurchmessern (17 nm, 45 nm, 81 nm, 124 nm) aufweist. Bereits nach wenigen Tagen erreichten die L929 Fibroblasten auf den Zellkultursubstraten gemäß der vorliegenden Erfindung ähnliche Proliferationsraten wie auf den glatten Kontrolloberflächen (Figur 3).

Somit können auf den Zellkultursubstraten gemäß der vorliegenden Erfindung mit topographischer Stimulation der Zellen durch die nanoporös strukturierte Oberfläche ähnliche Proliferationsraten erreicht werden wie beim Wachstum von Zellen auf Standard-2D-Oberflächen.

### 4. Proliferation von SK-MEL-28 Melanomzellen in Überkopfkultur auf Zellkultursubstraten gemäß der vorliegenden Erfindung im Vergleich zur Proliferation auf glatten Glasoberflächen

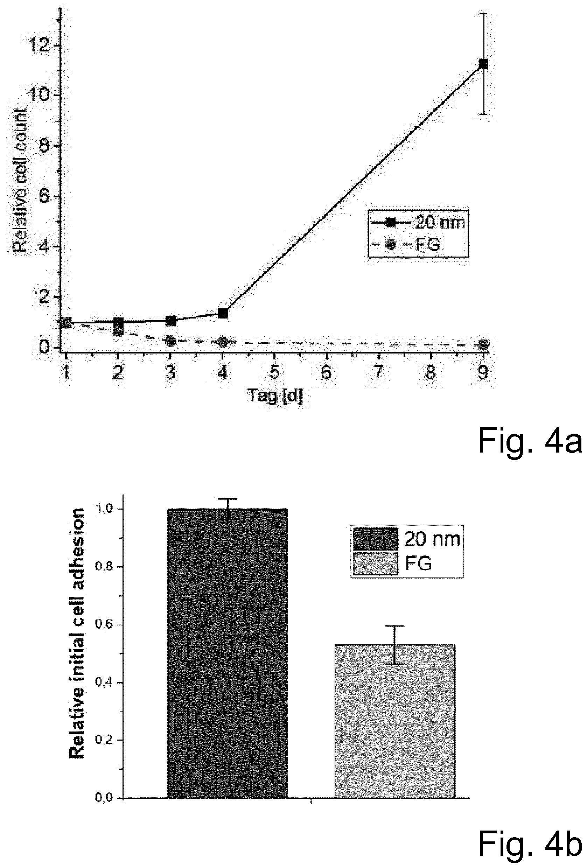

Zur Untersuchung der Proliferation von SK-MEL-28 Melanomzellen in Überkopfkultur auf den nanoporös strukturierten Oberflächen der Zellkultursubstrate gemäß der vorliegenden Erfindung im Vergleich zum Wachstum der Zellen auf glatten Glasoberflächen wurden SK-MEL-28 Melanomzellen auf den unterschiedlichen Substraten ausgesät und über einen Zeitraum von 9 Tagen in Überkopfkultur inkubiert. Dabei lässt sich für die Zellen, die auf den Zellkultursubstraten gemäß der vorliegenden Erfindung (Zellkultursubstrat mit nanoporöser VYCOR-Membran) mit einem durchschnittlichen Porendurchmesser von 20 nm inkubiert wurden, eine starke Proliferation in Überkopfkultur feststellen, während die Zellzahl auf den glatten Glasoberflächen unter den gleichen Bedingungen stetig abnimmt (Figur 4a).

Insbesondere konnte auch festgestellt werden, dass bereits nach 3 Stunden Inkubation auf einer flachen unporösen Glasoberfläche die Adhäsion von SK-MEL-28 Melanomzellen mit einer relativen Zellzahl von 0,53 ± 0,07 deutlich geringer ist als die Adhäsion von SK-MEL-28 Melanomzellen auf einer nanoporösen Glasmembran mit einem durchschnittlichen Porendurchmesser von 20 nm (Figur 4b).

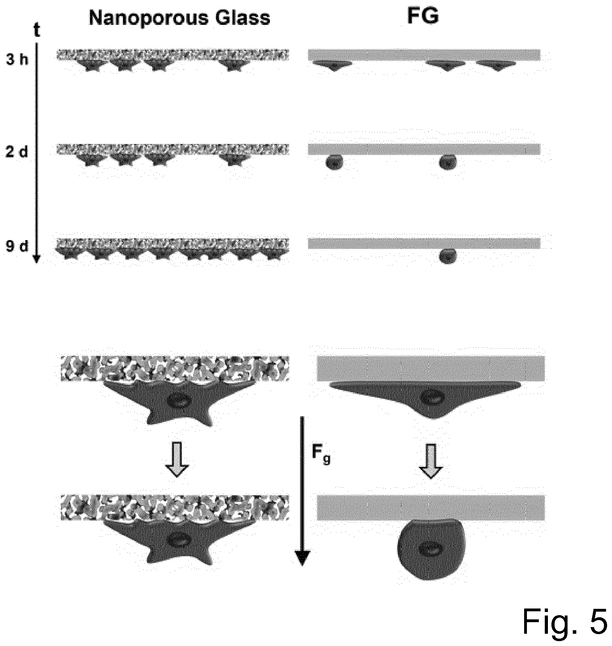

Zusätzlich konnte mittels Rasterelektronenmikroskopie gezeigt werden, dass die auf einer flachen unporösen Glasoberfläche gewachsenen Zellen signifikant häufiger eine runde Form und eine höhere Dichte aufweisen, was auf einen eher passiven Ausbreitungsprozess mit runderer Morphologie und weniger Filopodien hinweist. Im Gegensatz dazu hatten die auf nanoporösen Glasmembranen gewachsenen Zellen mehr Filopodien und nahmen einen weiteren Bereich der Substratoberfläche ein, was einen aktiven Ausbreitungsprozess mit starker fokalen Adhäsion der Zellen in Überkopfkultur an die topographische Oberfläche aufzeigt (Figur 5).

Demnach ist es mit Hilfe der Zellkultursubstrate gemäß der vorliegenden Erfindung vorteilhafterweise möglich, die Zelladhäsion durch die Simulierung einer dreidimensionalen Umgebung auch unter Wirkung der Schwerkraft und ohne zusätzliche Funktionalisierung/Beschichtung zu verbessern. Somit kommt die Oberfläche der Zellkultursubstrate gemäß der vorliegenden Erfindung der natürlichen Umgebung im menschlichen Körper deutlich näher als glatte 2D-Oberflächen.

### 5. Unterschiedliche mRNA-Expression auf nanoporösen Glasmembranen mit unterschiedlichem durchschnittlichen Porendurchmesser

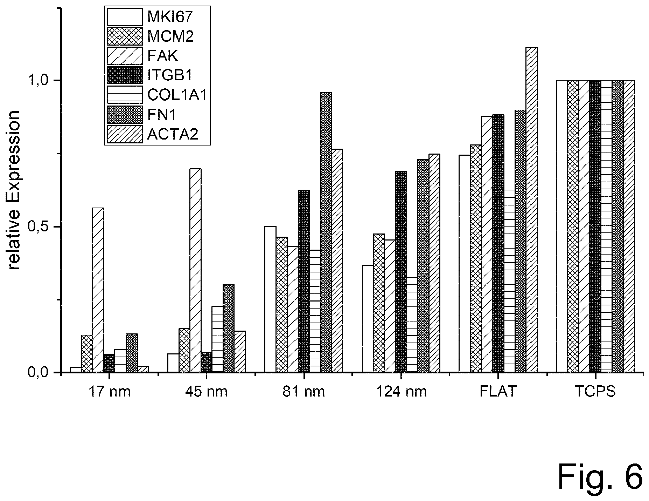

Die Analyse der mRNA-Expression von L929 Zellen auf den unterschiedlichen nanoporösen Glasmembranen (17 nm, 45 nm, 81 nm, 124 nm) mittels qPCR erfolgte nach 48 h Kultivierung, das heißt während der anfänglichen Ruhephase in der sich die Zellen auf der Oberfläche der Membranen absetzen (Figur 6). Es zeigt sich, dass insbesondere Zellen, die auf nanoporösen Glasmembranen mit einem durchschnittlichen Porendurchmesser von 81 nm beziehungsweise 124 nm kultiviert werden, ein mRNA-Expressionsprofil haben, das demjenigen von auf einer flachen unporösen Glasoberfläche kultivierten Zellen sehr ähnlich ist. Dies zeigt eine positive Interaktion zwischen den Zellen und der Oberfläche, obwohl zu diesem Zeitpunkt noch keine extensive Proliferation der Zellen begonnen hat. Zudem ist das Auslösen der Zellproliferation bei den nanoporösen Glasmembranen mit einem durchschnittlichen Porendurchmesser von 81 nm beziehungsweise 124 nm im Vergleich zu den anderen nanoporösen Glasmembranen signifikant erhöht. Das zeigt sich in der erhöhten Expression proliferationsspezifischer Proteine (MKI67, MCM2). Zusätzlich wurden auch Gene analysiert, die andere Zellfunktionen regulieren, wie die Zelladhäsion (FAK, Itgb1), Matrixproduktion (COL1A1, FN1) und Kontraktion (ACTA2). Auffällig ist hierbei die verringerte Expression von ACTA2 durch auf den nanoporösen Glasmembranen kultivierten Zellen im Vergleich zu auf der flachen unporösen Glasoberfläche kultivierten Zellen. Unterhalb einem durchschnittlichen Porendurchmesser von 80 nm ist eine drastische Änderung des Expressionsprofil feststellbar, wobei es in auf diesen nanoporösen Glasmembranen kultivierten Zellen zu einer deutlich erhöhten Expression von PTK2/FAK (Focal Adhesion Kinase) kommt, wohingegen andere essentielle Gene stark herunterreguliert sind.

### 6. Simulation des physiologischen Adhäsionsmechanismus von Zellen zum Wirknachweis Zytoskelett-wirksamer Wirkstoffe

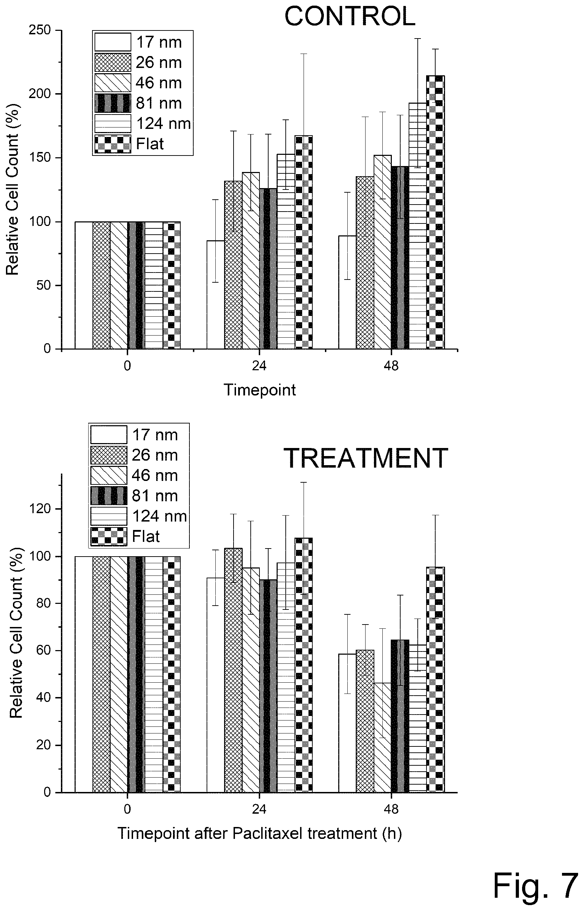

Im vorliegenden Versuch wurden MDA-MB-231 Brustkrebszellen zunächst auf Zellkultursubstraten gemäß der vorliegenden Erfindung, insbesondere nanoporösen Glasmembranen mit durchschnittlichen Porendurchmessern von 17 nm, 26 nm, 46 nm, 81 nm und 124 nm, und auf einer glatten unporösen Glasoberfläche ausgesät und für 24 h kultiviert, um eine gleichmäßige Zellbesiedelung auf allen Substraten zu erhalten. Anschließend wurden die Proben umgedreht und in zwei Gruppen aufgeteilt: eine Kontrollgruppe und eine Testgruppe, wobei die Kultivierung in Überkopfkultur für 48 h erfolgte. Dabei wurde die Kontrollgruppe in normalem Kulturmedium kultiviert und dem Kulturmedium der Testgruppe 500 nM Paclitaxel hinzugefügt. Während der 48-stündigen Kultivierung in Überkopfkultur konnte in der Testgruppe ein Rückgang der relativen Zellzahl auf den erfindungsgemäßen Substraten um ca. 35-55% im Vergleich zur Kontrollgruppe beobachtet werden (Figur 7). Interessanterweise fiel der Rückgang der relativen Zellzahl auf der glatten unporösen Glasoberfläche innerhalb der 48 h deutlich geringer aus (ca. 5%).

Das vorliegende Ergebnis zeigt die Möglichkeit der Simulation des physiologischen Adhäsionsmechanismus auf den Zellkultursubstraten gemäß der vorliegenden Erfindung und indiziert die Eignung der Zellkultursubstrate für den Wirknachweis aktiver Substanzen, die in zytoskelettale Vorgänge eingreifen.

### 7. Proliferation primärer humaner mesenchymaler Stammzellen (hMSC) auf nanoporösen Glasmembranen mit unterschiedlicher Porengröße

Primäre hMSC wurden auf nanoporösen Glasmembranen mit drei unterschiedlichen durchschnittlichen Porendurchmessern und zwei Kontrollsubstraten (TCPS = Tissue Culture Polysterene, FG = flaches Deckglas) ausgesät. Zu unterschiedlichen Zeitpunkten wurden die Proben mit Glutaraldehyd fixiert und für die Rasterelektronenmikroskopie vorbereitet. Alle untersuchten Proben zeigten eine gute Zelladhäsion und -proliferation. Während der ersten Tage der Kultivierung auf den nanoporösen Glasmembranen konnte die Bildung von Zellhaufen beobachtet werden. Diese waren nach Tag 3 nicht mehr vorhanden, was eine vollständige Ausbreitung der Zellen zeigt.

## Patentansprüche

1. Verfahren zur Kultivierung von Zellen, umfassend die Schritte:
a) Bereitstellen mindestens einer in einem Zellkulturmedium vorliegenden Zelle und eines Zellkultursubstrats,
b) Kontaktieren der mindestens einen in dem Zellkulturmedium vorliegenden Zelle mit dem Zellkultursubstrat,
c) Inkubieren der mindestens einen in dem Zellkulturmedium vorliegenden Zelle auf dem Zellkultursubstrat,
**dadurch gekennzeichnet, dass** das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

2. Verfahren nach Anspruch 1, wobei die mindestens eine in Schritt a) bereitgestellte in dem Zellkulturmedium vorliegende Zelle eine Stammzelle ist und das Verfahren ein Verfahren zur Differenzierung von Stammzellen ist.

3. Verfahren nach einem der Ansprüche 1 oder 2, **dadurch gekennzeichnet, dass** die nanoporös strukturierte Oberfläche einen durchschnittlichen Porendurchmesser von 40 bis 150 nm, bevorzugt 80 bis 150 nm, aufweist.

4. Verfahren nach Anspruch 2 oder 3, **dadurch gekennzeichnet, dass** dem Zellkulturmedium keine Additive zugesetzt werden.

5. Verfahren nach einem der vorherigen Ansprüche, **dadurch gekennzeichnet, dass** das Zellkultursubstrat eine Dicke von 10 bis 5000 µm aufweist.

6. Verfahren nach einem der vorherigen Ansprüche, **dadurch gekennzeichnet, dass** das Zellkultursubstrat transparent ist.

7. Verfahren nach einem der vorherigen Ansprüche, **dadurch gekennzeichnet, dass** das Zellkultursubstrat, insbesondere das Zellkultursubstrat aus Glas, eine Oberflächenfunktionalisierung und/oder Oberflächenbeschichtung aufweist.

8. Verfahren nach einem der vorherigen Ansprüche, **dadurch gekennzeichnet, dass** es sich bei dem Zellkultursubstrat um einen Teil eines Zellkulturgefäßes oder Bioreaktors handelt.

9. Verfahren nach einem der Ansprüche 1 bis 7, **dadurch gekennzeichnet, dass** es sich bei dem Zellkultursubstrat um einen Einsatz für Zellkulturgefäße oder Bioreaktoren handelt.

10. Verwendung eines Zellkultursubstrats zur Kultivierung oder Differenzierung von Zellen, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

11. Verwendung eines Zellkultursubstrats als Boden eines Zellkulturgefäßes oder Bioreaktors, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

12. Verwendung eines Zellkultursubstrats als herausnehmbarer Einsatz eines Zellkulturgefäßes oder Bioreaktors, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

13. Verwendung eines Zellkultursubstrats als perfusive Membran für 3D-Zellkultur-Reaktoren, wobei das Zellkultursubstrat ein Zellkultursubstrat aus Glas umfasst und mindestens ein Teil des Zellkultursubstrats aus Glas eine nanoporös strukturierte Oberfläche mit einem durchschnittlichen Porendurchmesser von 2 bis 150 nm aufweist.

## Claims

1. A method for cultivating cells, comprising the steps of:
a) providing at least one cell present in a cell culture medium, and a cell culture substrate,
b) contacting the at least one cell present in the cell culture medium with the cell culture substrate,
c) incubating the at least one cell present in the cell culture medium on the cell culture substrate,
**characterised in that** the cell culture substrate comprises a cell culture substrate of glass, and at least a part of the cell culture substrate of glass has a surface with a nanopore structure having an average pore diameter of 2 to 150 nm.

2. The method according to claim 1, wherein the at least one cell present in the cell culture medium that is provided in step a) is a stem cell, and the method is a method for differentiating stem cells.

3. The method according to any one of claims 1 or 2, **characterised in that** the surface with the nanopore structure has an average pore diameter of 40 to 150 nm, preferably 80 to 150 nm.

4. The method according to claim 2 or 3, **characterised in that** no additives are added to the cell culture medium.

5. The method according to any one of the preceding claims, **characterised in that** the cell culture substrate has a thickness of 10 to 5000 µm.

6. The method according to any one of the preceding claims, **characterised in that** the cell culture substrate is transparent.

7. The method according to any one of the preceding claims, **characterised in that** the cell culture substrate, in particular the cell culture substrate of glass, has a surface functionalization and/or a surface coating.

8. The method according to any one of the preceding claims, **characterised in that** the cell culture substrate is a part of a cell culture vessel or of a bioreactor.

9. The method according to any one of claims 1 to 7, **characterised in that** the cell culture substrate is an insert for cell culture vessels or bioreactors.

10. Use of a cell culture substrate for cultivating or differentiating cells, wherein the cell culture substrate comprises a cell culture substrate of glass, and at least a part of the cell culture substrate of glass has a surface with a nanopore structure having an average pore diameter of 2 to 150 nm.

11. Use of a cell culture substrate as bottom of a cell culture vessel or a bioreactor, wherein the cell culture substrate comprises a cell culture substrate of glass, and at least a part of the cell culture substrate of glass has a surface with a nanopore structure having an average pore diameter of 2 to 150 nm.

12. Use of a cell culture substrate as a removable insert for a cell culture vessel or a bioreactor, wherein the cell culture substrate comprises a cell culture substrate of glass, and at least a part of the cell culture substrate of glass has a surface with a nanopore structure having an average pore diameter of 2 to 150 nm.

13. Use of a cell culture substrate as a perfusive membrane for 3D cell culture reactors, wherein the cell culture substrate comprises a cell culture substrate of glass, and at least a part of the cell culture substrate of glass has a surface with a nanopore structure having an average pore diameter of 2 to 150 nm.

## Revendications

1. Procédé pour cultiver des cellules, comportant les étapes consistant à :
a) fournir au moins une cellule présente dans un milieu de culture cellulaire, et un substrat de culture cellulaire,
b) mettre en contact la au moins une cellule présente dans le milieu de culture cellulaire avec le substrat de culture cellulaire,
c) incuber la au moins une cellule présente dans le milieu de culture cellulaire sur le substrat de culture cellulaire,
**caractérisé en ce que** le substrat de culture cellulaire comprend un substrat de culture cellulaire en verre, et au moins une partie du substrat de culture cellulaire en verre a une surface de structure nanoporeuse avec un diamètre moyen de pore de 2 à 150 nm.

2. Procédé selon la revendication 1, dans lequel la au moins une cellule présente dans le milieu de culture cellulaire fournie dans l'étape a) est une cellule souche, et le procédé est un procédé pour différencier des cellules souches.

3. Procédé selon l'une quelconque des revendications 1 ou 2, **caractérisé en ce que** la surface de structure nanoporeuse a un diamètre moyen de pore de 40 à 150 nm, de préférence de 80 à 150 nm.

4. Procédé selon la revendication 2 ou 3, **caractérisé en ce qu'**aucun additif n'est ajouté au milieu de culture cellulaire.

5. Procédé selon l'une quelconque des revendications précédentes, **caractérisé en ce que** le substrat de culture cellulaire a une épaisseur de 10 à 5000 µm.

6. Procédé selon l'une quelconque des revendications précédentes, **caractérisé en ce que** le substrat de culture cellulaire est transparent.

7. Procédé selon l'une quelconque des revendications précédentes, **caractérisé en ce que** le substrat de culture cellulaire, notamment le substrat de culture cellulaire en verre, est pourvu d'une fonctionnalisation de surface et/ou d'un revêtement de surface.

8. Procédé selon l'une quelconque des revendications précédentes, **caractérisé en ce que** le substrat de culture cellulaire est un fond d'un récipient de culture cellulaire ou d'un bioréacteur.

9. Procédé selon l'une quelconque des revendications 1 à 7, **caractérisé en ce que** le substrat de culture cellulaire est un insert pour un récipient de culture cellulaire ou un bioréacteur.

10. Utilisation d'un substrat de culture cellulaire pour cultiver ou différencier des cellules, dans laquelle le substrat de culture cellulaire comporte un substrat de culture cellulaire en verre, et au moins une partie du substrat de culture cellulaire en verre a une surface de structure nanoporeuse avec un diamètre moyen de pore de 2 à 150 nm.

11. Utilisation d'un substrat de culture cellulaire en tant que fond d'un récipient de culture cellulaire ou d'un bioréacteur, dans laquelle le substrat de culture cellulaire comporte un substrat de culture cellulaire en verre, et au moins une partie du substrat de culture cellulaire en verre a une surface de structure nanoporeuse avec un diamètre moyen de pore de 2 à 150 nm.

12. Utilisation d'un substrat de culture cellulaire en tant qu'insert d'un récipient de culture cellulaire ou d'un bioréacteur, dans laquelle le substrat de culture cellulaire comporte un substrat de culture cellulaire en verre, et au moins une partie du substrat de culture cellulaire en verre a une surface de structure nanoporeuse avec un diamètre moyen de pore de 2 à 150 nm.

13. Utilisation d'un substrat de culture cellulaire en tant que membrane perfusive pour réacteurs de culture cellulaire en 3D, dans laquelle le substrat de culture cellulaire comporte un substrat de culture cellulaire en verre, et au moins une partie du substrat de culture cellulaire en verre a une surface de structure nanoporeuse avec un diamètre moyen de pore de 2 à 150 nm.
